# FASCICULE DE BREVET EUROPEEN

(11) **EP 1 396 242 B1**
(45) Date de publication et mention de la délivrance du brevet: **14.11.2007**
(21) Numéro de dépôt: 02019936.0
(22) Date de dépôt: 04.09.2002
(51) Int. Cl.: A61F 5/00, A61B 17/12

(54) **Système de fermeture pour anneau chirurgical**
Vorrichtung zum Verschliessen von chirurgischen Ringen
Closure system for surgical ring

(43) Date de publication de la demande: 10.03.2004
(73) Titulaire: EndoArt S.A., Ecublens (VD) (CH)
(72) Inventeur: Bachmann, Michel André, 1126 Vaux sur Morges (CH)
(74) Mandataire: Vossius & Partner

(56) Documents cités:
- WO-A-01/49245
- FR-A- 2 823 663
- US-A- 4 118 805

## Description

La présente invention se rapporte au domaine technique des implants chirurgicaux destinés à être implantés dans le corps d'un patient autour d'organe(s) biologique(s) constituant une poche ou un conduit, et plus particulièrement aux anneaux gastriques conçus pour traiter l'obésité par implantation d'un anneau gastrique souple, destiné à former une boucle fermée autour de l'estomac pour réduire le diamètre de l'ouverture du stoma.

La présente invention concerne un anneau chirurgical destiné à être implanté dans le corps d'un patient autour d'organe(s) biologique(s) constituant une poche ou un conduit pour modifier la section de passage dudit organe lorsqu'il est enserré par l'anneau, ledit anneau étant formé par une bande souple comprenant une première et une deuxième extrémités, ladite bande souple étant destinée à être fermée vers ses deux extrémités par un système de fermeture pour former une boucle close, ladite boucle close présentant une surface interne de contact avec l'organe biologique et une surface externe opposée.

L'invention concerne plus particulièrement un anneau de gastroplastie, mais elle peut aussi concerner un anneau conçu pour être utilisé pour traiter l'incontinence urinaire ou fécale (sphincter artificiel), ou encore un anneau conçu pour régler le débit sanguin dans des vaisseaux sanguins par exemple, cette liste n'étant nullement limitative.

Il est déjà connu d'intervenir de manière chirurgicale sur des patients atteints d'obésité extrêmement sévère (obésité morbide), c'est-à-dire dans le cas de patients dont le poids excède par exemple le poids idéal d'au moins 50 Kg, en implantant des anneaux de gastroplastie sur de tels patients. De telles interventions permettent d'éviter non seulement une série de problèmes de santé graves provenant d'un tel surpoids, mais encore et surtout d'éviter une mort certaine et proche de ces patients.

Il est en effet acquis que les patients souffrant d'obésité morbide voient leur espérance de vie réduite de manière importante, et d'au moins d'une dizaine à une quinzaine d'années, tout en souffrant d'importants problèmes de charge psychologique.

Par ailleurs, toute une série de phénomènes annexes de santé sont impliqués, ayant une incidence sur l'apparition de maladies annexes, telles que des maladies cardiovasculaires, l'hypertension, le diabète, ou encore des arthrites sévères notamment.

Il est également acquis que, pour de tels patients, les traitements basés sur des diètes sévères combinées à une série d'exercices physiques, associés également à une modification du comportement, notamment alimentaire, sont généralement peu adaptés, même si ces méthodes de traitement sont reconnues comme étant les plus saines.

C'est la raison pour laquelle les traitements efficaces et à long terme de l'obésité morbide font intervenir un traitement chirurgical.

De manière générale, on distingue des techniques de traitement chirurgical faisant intervenir un défaut d'absorption des aliments, c'est-à-dire un raccourcissement du passage des aliments et des sucs digestifs, et des techniques faisant intervenir une restriction gastrique réduisant la taille de l'estomac.

Les techniques chirurgicales impliquant un défaut d'absorption sont celles impliquant par exemple une technique de « by pass » ou de dérivation du petit intestin, ou encore celles mettant en oeuvre une séparation du passage des aliments relativement aux sucs digestifs.

Ces techniques chirurgicales sont relativement lourdes et peuvent donner lieu à de sévères complications, et c'est la raison pour laquelle elles ne sont plus guère utilisées maintenant.

On tend en effet désormais à utiliser des techniques chirurgicales qui mettent en oeuvre des interventions chirurgicales plus réduites, telles que la restriction gastrique impliquant la pose d'un anneau gastrique.

Ces techniques sont maintenant d'utilisation assez courante, et pour la plupart mettent en oeuvre, tel que décrit par exemple dans le document WO-94/27504, une bande souple destinée à être implantée autour de l'estomac, en formant une boucle fermée définissant un périmètre fixe préétabli de l'anneau, grâce à un système de fermeture. Le corps de la bande souple comporte, contre sa surface destinée à entrer en contact avec l'estomac, une chambre de gonflage à volume variable, qui est reliée à un cathéter de réglage permettant d'injecter ou de retirer un fluide dans la chambre de compression, de manière à faire varier le périmètre interne de la boucle pour modifier ou régler le diamètre du stoma. Ainsi, en combinaison avec le diamètre fixe et préétabli de l'anneau, on peut régler, dans une faible proportion, le diamètre de l'anneau, ce qui permet de réguler le diamètre du stoma, et donc de régler la quantité d'aliments ingérés.

Le système de fermeture décrit par le document WO-94/27504 met en oeuvre un fourreau solidaire d'une extrémité de la bande souple, l'ouverture dudit fourreau s'étendant tangentiellement à l'anneau et étant conformée pour coopérer avec une tête profilée solidaire de la deuxième extrémité de la bande souple, pour que ladite tête puisse se déformer pour passer dans l'ouverture du fourreau et venir se bloquer en butée contre les bords de celui-ci, réalisant ainsi le verrouillage de l'anneau en une boucle close.

Un tel système de fermeture s'avère peu pratique à mettre en oeuvre lors de l'opération de verrouillage de l'anneau autour de l'estomac du patient.

En effet, afin de mener à bien cette opération, le chirurgien se voit dans l'obligation d'effectuer des efforts de traction dans une direction tangentielle à l'anneau correspondant à la direction dans laquelle s'étend l'ouverture du fourreau, afin d'amener la tête en coopération avec le fourreau. Cette direction tangentielle d'effort correspond, pour le chirurgien, à la réalisation de manipulations sensiblement contre et parallèlement à la paroi de l'estomac, ce qui s'avère être malaisé pour le praticien et risqué pour le patient, puisque cela augmente le risque pour le praticien d'endommager accidentellement l'estomac.

De plus, le chirurgien se voit contraint, pour procéder au verrouillage de l'anneau, de bloquer l'extrémité de la bande souple portant le fourreau, et de rapprocher de cette extrémité la deuxième extrémité portant la tête, puis d'engager cette dernière dans le fourreau en tirant directement sur la deuxième extrémité, à la manière d'un noeud coulant, jusqu'à ce que le verrouillage s'opère par emboîtement souple.

Le risque existe ainsi que le chirurgien exerce un effort de traction trop important sur la deuxième extrémité qui conduisent à l'endommagement de la bande souple constituant l'anneau.

Les inconvénients mentionnés précédemment sont exacerbés par le fait que la tête profilée est prolongée par le cathéter d'ajustement du diamètre, ce qui complique encore la gestuelle de fermeture, et peut conduire à un endommagement dudit cathéter.

Il s'avère également que les dispositifs de l'art antérieur, tel que celui décrit dans la demande WO-94/27504, souffrent d'inconvénients liés à la difficulté de réaliser toute intervention chirurgicale susceptible de survenir après la pose de l'implant de gastroplastie.

En effet, il s'avère que malgré la possibilité de modifier dans une certaine mesure le diamètre de l'anneau sans intervention chirurgicale, la pose de tels implants gastriques peut parfois s'accompagner de phénomènes d'intolérance, par exemple accompagnés de vomissements liés à une trop forte réduction du diamètre du stoma, ou encore une action inefficace de l'implant liée à un diamètre du stoma trop important, ou encore tout simplement à une gène ou une infection ou inflammation locale ou générale.

C'est la raison pour laquelle il est dans ce cas nécessaire d'intervenir à nouveau de manière chirurgicale, soit pour soulager le patient, soit pour modifier ou changer l'anneau de gastroplastie préalablement implanté.

De telles interventions chirurgicales sont particulièrement complexes et nécessitent l'ouverture complète de l'anneau, en vue de son changement et de son remplacement.

Les systèmes de fermeture de l'art antérieur n'autorisent pas une ouverture aisée de l'anneau, pour des raisons similaires à celles conduisant aux inconvénients rencontrés lors de la fermeture de l'anneau.

Dès lors, de telles interventions chirurgicales post-implantation conduisent souvent à la découpe de l'anneau par le chirurgien, en vue de son changement et de son remplacement.

En définitive, le verrouillage ou le déverrouillage des anneaux de l'art antérieur s'effectue donc en exerçant un effort d'ouverture ou de fermeture, du genre effort de traction, directement sur la bande souple elle-même. De tels efforts, exercés généralement à l'aide d'instruments contendants, du genre pinces chirurgicales, peuvent occasionner des dégâts à l'anneau (déchirement, percement...), qui peuvent être de nature rédhibitoire pour son fonctionnement.

Le risque d'occurrence de tels problèmes est accentué par la gestuelle d'ouverture / fermeture malcommode nécessitée par les dispositifs de l'art antérieur.

Par ailleurs, la mise en oeuvre d'un système de fermeture / ouverture de l'art antérieur est particulièrement délicate, voire impossible, dans le cas d'un anneau dont le diamètre est commandé par un dispositif d'actionnement mettant en oeuvre un actionneur, du genre moteur électrique, disposé à une extrémité de la bande souple formant l'anneau. La rigidité du bloc moteur et son encombrement relatif interdisent en effet tout principe d'ouverture /fermeture basé sur un enfilement d'une extrémité de la bande souple dans l'autre extrémité, et/ou un accouplement souple d'une extrémité dans l'autre.

L'invention vise en conséquence à porter remède aux différents inconvénients énumérés précédemment, et à proposer un nouvel anneau chirurgical, notamment gastrique, présentant un système de fermeture dont la mise en oeuvre réduit les contraintes mécaniques appliquées à la bande souple formant l'anneau lors de la pose et la dépose de l'anneau, et qui soit particulièrement pratique et sûr.

Un autre objet de l'invention vise à proposer un nouvel anneau chirurgical, notamment gastrique, présentant un système de fermeture qui soit particulièrement simple et bon marché.

Un autre objet de l'invention vise à proposer un nouvel anneau chirurgical, notamment gastrique, présentant un système de fermeture particulièrement efficace.

Un autre objet de l'invention vise à proposer un nouvel anneau chirurgical, notamment gastrique, susceptible de faciliter la manipulation de l'anneau lors de sa pose, ainsi que lors de la ré-ouverture et de la re-fermeture éventuelles de l'anneau.

Un autre objet de l'invention vise à proposer un nouvel anneau chirurgical, notamment gastrique, présentant un système de fermeture particulièrement fiable.

Un autre objet de l'invention vise à proposer un nouvel anneau chirurgical, notamment gastrique, qui soit particulièrement atraumatique et bien supporté par le patient.

Un autre objet de l'invention vise à proposer un nouvel anneau chirurgical, notamment gastrique, qui soit particulièrement robuste, compact et facile à fabriquer.

Un autre objet de l'invention vise à proposer un nouvel anneau chirurgical, notamment gastrique, dont le système de fermeture est particulièrement bien adapté à la présence d'un actionneur destiné à générer une variation du périmètre interne de l'anneau et monté à l'une des extrémités de la bande souple constituant ledit anneau.

Les objets assignés à l'invention sont atteints à l'aide d'un anneau chirurgical destiné à être implanté dans le corps d'un patient autour d'organe(s) biologique(s) constituant une poche ou un conduit pour modifier la section de passage dudit organe lorsqu'il est enserré par l'anneau, ledit anneau étant formé par une bande souple comprenant une première et une deuxième extrémités, ladite bande souple étant destinée à être fermée vers ses deux extrémités par un système de fermeture pour former une boucle close, ladite boucle close présentant une surface interne de contact avec l'organe biologique et une surface externe opposée, caractérisé en ce que le système de fermeture comprend un moyen d'encerclement solidaire de la première extrémité et agencé pour évoluer entre :
- une configuration de déverrouillage où le moyen d'encerclement forme un collier ouvert libérant la deuxième extrémité,
- et une configuration de verrouillage où le moyen d'encerclement forme un collier fermé destiné à entourer la deuxième extrémité en vue de la solidariser à la première extrémité, ledit collier fermé présentant une face avant et une face arrière opposées, entre lesquelles s'étend une ouverture d'encerclement destinée à accueillir la deuxième extrémité.

D'autres objets et avantages de l'invention apparaîtront mieux à la lecture de la description ci-jointe, ainsi qu'à l'aide des dessins annexés, à titre purement illustratif et informatif, dans lesquels :
- La figure 1 représente, selon une vue en perspective, un exemple de réalisation d'un anneau chirurgical conforme à l'invention en position ouverte.
- La figure 2 représente, selon une vue en perspective, l'anneau chirurgical représenté à la figure 1 en position fermée et équipé d'un actionneur.
- La figure 3 représente, selon une vue en perspective, l'anneau gastrique représenté aux figures 1 et 2, ledit anneau étant équipé d'une antenne de réception en vue de commander à distance l'actionneur.
- La figure 4 représente, selon une vue en perspective, un détail de l'actionneur de l'anneau représenté à la figure 3.
- La figure 5 représente, selon une vue en coupe longitudinale, l'anneau représenté aux figures 3 et 4.

Dans la description qui suit, il sera fait référence, uniquement à titre d'exemple, à un anneau gastrique conçu pour être implanté autour de l'estomac pour réduire le diamètre de l'ouverture du stoma ou autour de l'oesophage.

En effet, l'invention n'est nullement limitée à cette application, et vise au contraire à couvrir d'autres anneaux chirurgicaux, tels ceux utilisés pour traiter l'incontinence urinaire ou fécale, ou ceux utilisés autour de vaisseaux sanguins pour régler le débit sanguin.

Dans le cas de traitement d'incontinence urinaire, l'anneau sera implanté autour de la vessie ou des voies urinaires, et dans le cas de traitement d'incontinence fécale, il sera implanté autour des voies gastro-intestinales et notamment autour des structures anales de l'intestin.

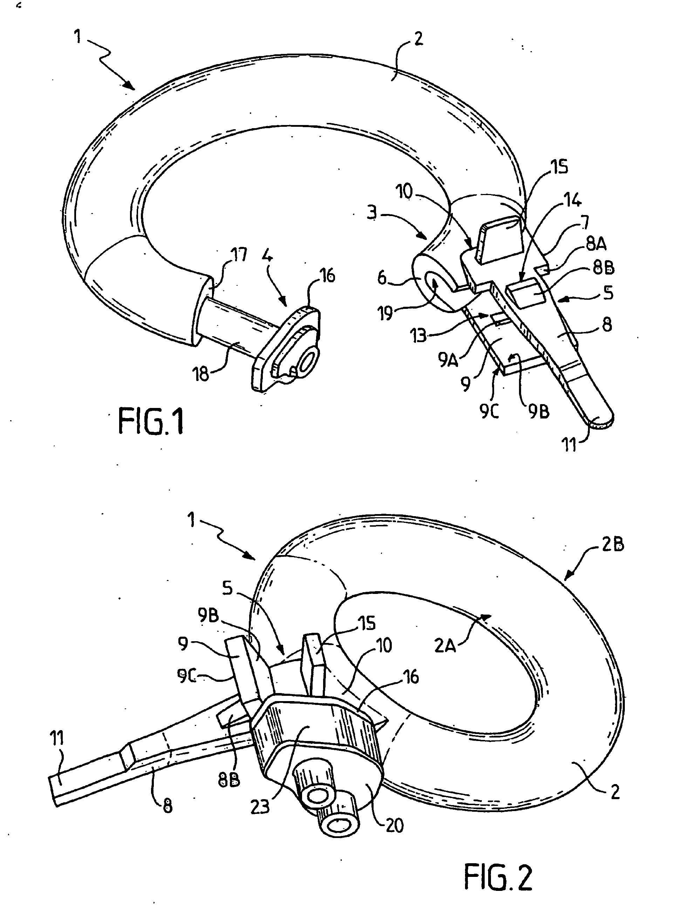

Les figures 1 à 5 illustrent un anneau gastrique 1 conforme à l'invention destiné à être implanté dans le corps d'un patient autour de l'estomac, pour modifier la section de passage de l'estomac, c'est-à-dire réaliser une restriction gastrique en réduisant le diamètre de l'ouverture du stoma lorsqu'il est enserré par l'anneau.

L'anneau gastrique 1 conforme à l'invention se présente sous la forme d'une bande souple 2, de préférence tubulaire, dont l'enveloppe flexible et élastique présente une surface lisse 2A, 2B, de manière à être facilement supportée par le patient et les tissus de l'estomac. La bande 2 est réalisée par exemple en matériau élastomère, du genre silicone.

La bande souple 2 comprend une première extrémité 3 et une deuxième extrémité 4. La bande souple 2 est destinée à être fermée vers ses deux extrémités 3, 4 par un système de fermeture pour former une boucle close, tel que cela est représenté aux figures 2 à 5.

Dans la variante de réalisation représentée aux figures, l'anneau gastrique 1 conforme à l'invention se présente sous la forme d'un tore de révolution, de section par exemple sensiblement circulaire, délimité extérieurement par une enveloppe monocouche ou multicouche qui peut être avantageusement formée d'un revêtement de protection, par exemple à base de ou en silicone.

La boucle close formée par la bande souple 2 présente une surface interne 2A de contact avec l'organe biologique, en l'occurrence l'estomac, et une surface externe opposée 2B.

Selon l'invention, le système de fermeture de la bande souple 2 comprend un moyen d'encerclement 5 monté solidaire de la première extrémité 3. Ledit moyen d'encerclement 5 est implanté et conformé sur ladite première extrémité 3 pour évoluer entre :
- une configuration de déverrouillage (cf. figure 1), où le moyen d'encerclement 5 forme un collier ouvert libérant la deuxième extrémité, c'est-à-dire n'ayant aucune interaction de maintien avec ladite deuxième extrémité,
- une configuration de verrouillage (cf. figure 2), où le moyen d'encerclement 5 forme un collier fermé destiné à entourer la deuxième extrémité en vue de la solidariser à la première extrémité, ledit collier fermé présentant une face avant 6 et une face arrière 7 opposées, entre lesquelles s'étend une ouverture d'encerclement destinée à accueillir la deuxième extrémité 4 de l'anneau.

Avantageusement, le moyen d'encerclement 5 est agencé pour passer de manière réversible entre la configuration de déverrouillage représentée à la figure 1 et la configuration de verrouillage représentée aux figures 2 à 5.

De cette façon, l'utilisateur peut ouvrir ou fermer l'anneau à volonté, sans altérer aucunement la fonctionnalité de celui-ci.

Il est cependant tout à fait envisageable que le moyen d'encerclement ne soit conçu que pour un usage unique, et qu'il ne puisse par exemple passer qu'une seule fois de la position de déverrouillage à la position de verrouillage, l'opération inverse nécessitant une altération plus ou moins partielle de l'anneau 1, sans pour autant sortir du cadre de l'invention.

Avantageusement, le moyen d'encerclement 5 comprend un élément mâle 8 et un élément femelle 9, tous deux montés solidaires de la première extrémité 3 de l'anneau, et montés sur ou relativement à cette dernière de telle façon que, lors de leur connexion mutuelle, le moyen d'encerclement 5 est verrouillé en formant le collier fermé.

Le moyen d'encerclement 5 permet ainsi de sangler la deuxième extrémité 4 à la première extrémité 3, à la façon d'une bride. On comprend dès lors que le concept général de l'invention s'oppose au concept de verrouillage développé dans l'art antérieur.

En effet, dans l'art antérieur, les moyens actifs de verrouillage étaient répartis sur chaque extrémité de la bande souple, de sorte que les efforts à appliquer pour le verrouillage ou le déverrouillage s'exerçait directement sur la bande souple, avec tous les inconvénients que cela peut engendrer.

Au contraire, l'invention permet d'effectuer le verrouillage de l'anneau sans exercer d'efforts sensibles de traction sur la bande souple, puisque les éléments principaux du système de verrouillage nécessitant une activation externe sont tous disposés sur la même extrémité de la bande souple, et ne sont pas répartis entre les deux extrémités.

De façon préférentielle, l'élément femelle 9 comprend un orifice 9A traversant l'épaisseur dudit élément femelle 9 entre une première face 9B et une deuxième face 9C opposée. L'élément mâle 8 comprend quant à lui, de façon préférentielle, une languette destinée à être enfilée dans l'orifice 9, ladite languette étant pourvue de moyens de blocage 8A, 8B agencés pour coopérer avec l'élément femelle 9, et plus particulièrement avec l'orifice 9A.

De façon préférentielle, la languette 8 s'étend à partir de la surface extérieure de l'anneau au niveau de la première extrémité 3, de manière sensiblement longiligne, et présente ainsi une extrémité de liaison 10 rattachée à la surface externe de l'anneau 1, et une extrémité libre 11.

L'élément femelle 9 est également rattaché à la surface externe de l'anneau, en regard de la languette 8, et s'étend de façon sensiblement longiligne parallèlement à la languette 8, et est séparé de cette dernière par le diamètre, ou l'épaisseur, de la bande souple 2.

De façon préférentielle, la languette 8 et l'élément femelle 9 sont orientés pour que le collier fermé s'étende vers l'extérieur de l'anneau 1, de façon à déporter le système de fermeture afin d'en faciliter la manipulation par le praticien.

L'ouverture d'encerclement définie par le collier fermé appartient ainsi à un plan qui, de façon préférentielle, forme un angle sensiblement oblique par rapport à la direction radiale correspondante, étant entendu qu'il est tout à fait envisageable que ce dit plan s'étende radialement par rapport à l'axe de symétrie principal de l'anneau.

De façon préférentielle, la languette 8 comprend d'une part un premier moyen d'appui 8A formant premier moyen de blocage, et destiné à venir en appui contre le bord périphérique 13 de l'orifice 9A sur la première face 9B de l'élément femelle 9. La languette 8 comprend d'autre part un deuxième moyen d'appui 8B formant deuxième moyen de blocage, et destiné à venir en appui contre le bord périphérique de l'orifice 9A sur la deuxième face 9C de l'élément femelle 9. Le deuxième moyen d'appui 8B est conformé pour coopérer avec l'orifice 9A, à la manière d'une came avec une butée. En d'autres termes, le deuxième moyen d'appui 8B est agencé pour pouvoir passer complètement à travers l'orifice 9A, tout en étant doté d'un moyen anti-retour l'empêchant de faire spontanément le chemin inverse, réalisant ainsi le verrouillage de l'anneau. Ce système came-butée peut présenter un caractère réversible, c'est-à-dire autoriser la désactivation du moyen anti-retour de la came constitué par le moyen d'appui 8B. Les premier et deuxième moyens d'appui 8A, 8B sont agencés de façon préférentielle l'un relativement à l'autre pour venir, en configuration de verrouillage (représentée aux figures 2 à 5), enserrer entre eux l'élément femelle 9, de façon à assurer ainsi une configuration de verrouillage stable, où sensiblement aucune variation du périmètre interne du collier fermé n'est autorisée.

De façon préférentielle, la languette 8 et l'élément femelle 9 sont formés tous deux d'une lamelle souple et réalisés en un matériau flexible du genre élastomère.

Ladite languette 8, tel que cela est représenté aux figures, s'étend à partir de son extrémité de liaison 10 selon une forme sensiblement en T, la jonction entre la petite branche et la grande branche du T correspondant à un épaulement, lequel épaulement définit une surface d'appui 8A formant premier moyen d'appui, la grande branche du T étant préférentiellement profilée en biseau pour faciliter son introduction et son passage dans l'orifice 9A. La partie de la languette 8 formant la grande branche du T comprend également une excroissance souple 8B formant deuxième moyen d'appui. Cette excroissance souple 8B présente une surface inclinée formant rampe permettant de faciliter son introduction dans l'orifice 9A, ladite surface inclinée étant interrompue par une surface plane 14 sensiblement perpendiculaire au corps de la languette 8, ladite surface plane faisant office de deuxième moyen d'appui.

De façon préférentielle, l'extrémité libre 11 de la languette 8 est conformée pour faire office de première patte de préhension, ladite première patte de préhension permettant à l'utilisateur, en la saisissant, de faire passer la languette 8 et l'excroissance souple 8B par l'orifice 9A en vue de former le collier fermé.

De façon avantageuse, l'anneau selon l'invention comporte une deuxième patte de préhension 15 permettant à l'utilisateur de maintenir l'élément mâle formé par la languette 8 lorsqu'il manipule l'élément femelle 9, en vue de procéder à la séparation des éléments mâle et femelle afin d'ouvrir l'anneau 1. Ladite deuxième patte de préhension 15 s'étend de manière préférentielle sensiblement vers l'extérieur de l'anneau 1 perpendiculairement à la languette 8 et à partir de celle-ci, à proximité de l'extrémité de liaison 10.

L'élément femelle 9 comprend avantageusement une troisième patte de préhension permettant de faciliter la séparation des éléments mâle 8 et femelle 9, en vue d'ouvrir l'anneau 1. De façon préférentielle, tel que cela est représenté aux figures, le corps de l'élément femelle 9 est conformé pour former lui-même ladite troisième patte de préhension.

Le système mâle-femelle de verrouillage décrit précédemment n'est bien sûr pas le seul envisageable dans le cadre de l'invention, et on pourra lui substituer tout autre moyen de fermeture bien connu de l'homme du métier, et mettant par exemple en oeuvre une fermeture à grenouillère, des bandes Velcro®, ou encore une fermeture de type ceinture de pantalon, sans pour autant sortir du cadre de l'invention.

Par ailleurs, si l'on a décrit en détail jusqu'à présent des moyens d'encerclement comprenant deux brins connectables pour former un collier fermé, il est envisageable, sans pour autant sortir du cadre de l'invention, que le moyen d'encerclement soit formé d'un collier conservant une forme de boucle en permanence, mais dont le périmètre interne soit réglable par tout moyen connu, pour notamment atteindre une dimension suffisamment importante par rapport à la dimension de la section transversale de la deuxième extrémité 4, de façon à libérer cette deuxième extrémité 4 de toutes contraintes mécaniques, notamment de frottement ou de serrage.

De façon avantageuse, la deuxième extrémité 4 de l'anneau 1 est pourvue d'un premier moyen d'arrêt 16 destiné à venir en butée contre la face arrière 7 du collier fermé entourant la deuxième extrémité 4 de l'anneau, lorsque ce dernier est en configuration de verrouillage, de façon à empêcher le déplacement, par coulissement ou glissement, de la deuxième extrémité 4 dans le sens de l'ouverture de l'anneau 1, c'est-à-dire dans le sens d'un accroissement du périmètre interne dudit anneau 1.

Avantageusement, la deuxième extrémité 4 de l'anneau 1 est pourvue également d'un deuxième moyen d'arrêt 17, destiné à venir en butée contre la face avant 6 du collier fermé entourant la deuxième extrémité 4 de l'anneau en configuration de verrouillage, de façon à empêcher le déplacement de la deuxième extrémité 4 dans le sens de la fermeture de l'anneau 1, c'est-à-dire dans le sens d'une restriction du périmètre interne dudit anneau 1.

De façon préférentielle, lesdits premier et deuxième moyens d'arrêt 16, 17 sont agencés l'un relativement à l'autre le long de la portion terminale de l'anneau 1 correspondant à la deuxième extrémité 4, pour enserrer entre eux le collier fermé en configuration de verrouillage, de façon à sensiblement empêcher tout déplacement de la deuxième extrémité 4 relativement à la première extrémité.

La combinaison du moyen d'encerclement 5 et des premier et deuxième moyens d'arrêt 16, 17 permet ainsi de solidariser la première extrémité 3 à la deuxième extrémité 4, à la fois du point de vue radial et du point de vue tangentiel, relativement à l'axe de symétrie principal de l'anneau 1.

Avantageusement, la bande souple 2 présente une portion de section réduite 18 au niveau de la deuxième extrémité 4 de l'anneau 1, ladite portion étant destinée à venir se loger latéralement dans une échancrure 19B, de forme sensiblement complémentaire, ménagée au niveau de la première extrémité 3 au sein du corps de l'anneau 1. Ladite échancrure 19B est positionnée dans la continuité des éléments mâle 8 et femelle 9, de façon à former sensiblement un U, où les bras du U sont formés respectivement par la languette 8 et l'élément femelle 9, tandis que l'âme du U est sensiblement constituée de l'échancrure 19B. Ladite échancrure forme ainsi une partie du collier fermé en configuration de verrouillage, ce qui permet d'assurer une continuité de la surface interne 2A de l'anneau lorsque celui-ci est en position fermée, tel que cela est représenté à la figure 2. La surface interne 2A de l'anneau 1 est ainsi régulière et continue, et ne présente aucune aspérité ou décrochement au niveau de la jonction des extrémités 3 et 4, ce qui garantit à l'anneau un caractère parfaitement atraumatique.

Avantageusement, la bande souple 2 présente un épaulement 17 au niveau de la transition à la portion de section réduite 18, ledit épaulement 17 faisant office de deuxième moyen d'arrêt. Ainsi, l'anneau selon l'invention présente un caractère particulièrement compact en position fermée, tel que l'on peut le constater à la figure 2.

De façon préférentielle, la bande souple 2 et le système de fermeture 5 forment une pièce en deux composants réalisés à partir de matériaux élastomères de dureté différente de manière à les adapter aux contraintes mécaniques spécifiques qu'elles subissent au cours de l'ouverture /fermeture du système.

A titre de variante, la bande souple 2 et le système de fermeture 5 peuvent former une pièce monobloc, avantageusement réalisée dans un même matériau.

Avantageusement, et conformément aux variantes de réalisation présentées aux figures, l'anneau 1 selon l'invention comprend un système pour commander de manière réversible la variation de son périmètre interne (correspondant à la surface interne 2A), ledit système comportant un élément souple filiforme 19 présentant une bonne flexibilité et une bonne résistance mécanique, inséré longitudinalement et à coulissement selon l'axe principal de symétrie du cylindre ou du corps principal de l'anneau 1, ledit élément 19 occupant la cavité reliant les première et deuxième extrémités 3, 4, et s'étendant sensiblement entre lesdites première et deuxième extrémités 3, 4, c'est-à-dire sensiblement sur toute la longueur développée de l'anneau 1.

Tel qu'illustré à la figure 5, l'élément souple filiforme 19 est monté pour définir ainsi une portion fixe 19A qui est solidarisée à l'aide de moyens de solidarisation 22, faisant par exemple intervenir un circlips et une rondelle, ou tout moyen équivalent, avec la première extrémité 3 de l'anneau 1. L'autre portion terminale de l'élément souple filiforme 4 forme une portion libre 21, c'est-à-dire susceptible de se déplacer par translation relativement à la portion fixe 19A, ladite portion libre 21 étant associée fonctionnellement à un actionneur 23 monté sur l'anneau 1 même vers ou au niveau de la deuxième extrémité 4. L'actionneur 23 est chargé de transmettre l'énergie nécessaire pour assurer, lorsqu'il est activé, la translation réversible de l'élément souple filiforme 19 à l'intérieur de l'anneau, i.e. le déplacement réversible de la portion libre 21 relativement à la portion fixe 19A, en vue d'obtenir une variation associée du périmètre interne de l'anneau 1, c'est-à-dire une augmentation ou une réduction de son diamètre interne.

Le montage direct de l'actionneur 23 sur l'une des extrémités 4 de l'anneau permet ainsi un gain de place important et une bonne efficacité mécanique.

Avantageusement, la portion libre 21, s'étendant par exemple sur une longueur de l'ordre de quelques centimètres ou sur toute la longueur de l'élément souple filiforme, est pourvue de moyens de coopération de force avec l'actionneur 23, lesdits moyens de coopération de force étant destinés à assurer la transmission de l'énergie fournie par l'actionneur 23 à l'ensemble de l'élément souple filiforme 19 à partir de son point d'appui matérialisé par la portion fixe 19A.

Avantageusement, les moyens de coopération de force sont formés par un pas de vis.

Selon l'invention, l'élément souple filiforme 19 présente une flexibilité suffisante pour pouvoir s'adapter à la forme sensiblement circulaire de l'anneau, tout en étant capable de transmettre la force nécessaire au réglage du diamètre de l'anneau. Avantageusement, l'élément souple filiforme 19 est formé par une âme souple, de préférence métallique, par exemple de section circulaire, sur laquelle est fixé et enroulé coaxialement, par exemple sur toute sa longueur, au moins un ressort à spires non jointives formant le pas de vis.

De manière particulièrement avantageuse, l'élément souple filiforme 19 comporte deux ressorts à spires non jointives pour former le pas de vis, respectivement un premier ressort enroulé hélicoïdalement le long de l'âme souple et un second ressort de diamètre extérieur supérieur, et comportant préférentiellement des spires de section transversale rectangulaire, de manière à délimiter une génératrice externe plane, ledit premier ressort étant interposé entre les spires du second ressort pour maintenir un pas de vis carré constant.

Grâce à cette disposition, il est ainsi possible de conserver constamment un pas sensiblement constant et efficace, même en cas de déformation de l'élément souple filiforme 19. Ceci confère au dispositif une grande précision et une grande efficacité, tout en étant économe en énergie nécessaire à son fonctionnement en raison du rendement élevé de la transmission par une vis à pas carré.

Grâce à cette disposition, il est possible de garantir une position de réglage stable même lorsque aucune énergie n'est fournie au système.

Le second ressort peut être avantageusement obtenu par découpage laser d'un tube cylindrique creux, son montage sur et entre les spires du premier ressort étant effectué après une traction longitudinale. Le second ressort est donc animé naturellement d'une force élastique intrinsèque de compression tendant à rendre les spires jointives, cette force intrinsèque étant contrecarrée par les spires du premier ressort, contre lesquelles elles viennent en appui. On bénéficie ainsi d'un pas constant malgré l'élasticité et la flexibilité naturelles et indispensables de l'élément souple allongé.

L'actionneur 23 pourra être formé par tout moyen classique bien connu de l'homme du métier susceptible de coopérer avec le pas de vis pour lui transmettre un mouvement. De manière particulièrement avantageuse, l'actionneur 23 pourra être pourvu d'un simple écrou permettant d'assurer l'entraînement du pas de vis, l'actionneur 23 pouvant être de manière générale un moyen moteur, du genre moteur électrique, électromagnétique ou autre, sans pour autant sortir du cadre de l'invention.

De manière préférentielle, l'actionneur est monté sur l'anneau 1 pour constituer le premier moyen d'arrêt 16, ou du moins être associé à celui-ci.

De façon préférentielle, tel que cela est représenté aux figures, l'actionneur est monté entre une première et une deuxième flasques parallèles 16, 20 (la deuxième flasque 20 n'est pas illustrée aux figures 1, 4 et 5), ladite première flasque 16 constituant le premier moyen d'arrêt.

A titre de variante de réalisation non représentée aux figures, il est bien évidemment possible de remplacer le pas de vis décrit précédemment par tout moyen technique équivalent, et par exemple par une crémaillère engrenant sur un actionneur 23 pourvu d'une roue dentée ou d'un moyen équivalent. On peut également envisager de réaliser l'élément souple filiforme 19 sous la forme d'un simple câble, entraîné de manière réversible par un actionneur 23 intégrant une poulie.

Tel qu'illustré aux figures, l'anneau gastrique 1 conforme à l'invention est, de manière générale, formé par un corps principal à base d'un matériau compressible 24 formant le matériau de base et qui vient remplir l'intérieur de l'enveloppe. Dans le matériau compressible 24 est inséré longitudinalement et sensiblement avec possibilité de coulissement, l'élément souple filiforme 19, tel qu'illustré par exemple à la figure 5.

De manière particulièrement avantageuse, le matériau compressible 24 est de l'ePTFE, dont les caractéristiques de compressibilité et de stabilité à la striction conviennent particulièrement bien à ce genre d'application.

Selon une variante de réalisation non illustrée aux figures, l'anneau conforme à l'invention comprend une enveloppe en matériau silicone et d'épaisseur sensiblement constante qui forme le revêtement extérieur étanche de l'anneau, l'intérieur de l'anneau étant formé exclusivement du matériau compressible 24, par exemple de l'ePTFE, à l'intérieur duquel l'élément souple filiforme 19 est inséré avec un léger jeu.

La première extrémité 3 comporte une poche, par exemple remplie de colle, et dans laquelle est montée et fixée la portion fixe 19A, avec les moyens de solidarisation 22.

Selon cette première variante de réalisation, l'action de l'actionneur 23 sur l'élément souple filiforme 19, transmet une force d'actionnement selon l'une des directions indiquées par la flèche F illustrée à la figure 5, ce qui a pour conséquence de comprimer ou relâcher, d'une manière sensiblement longitudinale, le matériau compressible 24 se traduisant par une variation associée du diamètre de l'anneau, tant interne qu'externe, sensiblement à la manière d'un noeud coulant.

Une autre variante de réalisation illustrée à la figure 5, ne diffère de la précédente que par l'agencement spécifique de l'enveloppe externe dont la périphérie dorsale 25 est renforcée en vue de brider l'extension radiale externe ou centrifuge de l'anneau pour, au contraire, privilégier la variation radiale interne ou centripète du diamètre de l'anneau. De cette manière, on privilégie la variation radiale du diamètre de l'anneau à sa périphérie interne qui est opposée à sa périphérie dorsale.

Telle qu'illustrée, la périphérie dorsale renforcée 25 peut être réalisée sous la forme d'une enveloppe externe dont seule la périphérie dorsale présente une surépaisseur, c'est-à-dire une épaisseur dorsale externe plus importante que le reste de l'enveloppe externe (figure 5). Alternativement ou de manière complémentaire, la périphérie dorsale 25 peut être également réalisée en utilisant un matériau polymère de dureté supérieure à la dureté du reste de l'enveloppe en matériau polymère. Il est également envisageable d'intégrer dans la périphérie dorsale renforcée 25 un insert de renfort, de préférence métallique, s'étendant sur la majorité de la périphérie de l'anneau entre le matériau compressible 24 et la périphérie dorsale 25. Avantageusement, l'insert peut présenter une mémoire de forme sensiblement circulaire pour obtenir une position de repos élastique circulaire de l'anneau.

Grâce à cet agencement, l'augmentation ou la réduction du diamètre de l'anneau est limitée à un déplacement radial réversible, situé au niveau de la périphérie interne de l'anneau opposé à la périphérie dorsale, se traduisant par une variation du diamètre interne de l'anneau en direction centrifuge ou centripète, selon le sens de la sollicitation imprimée à l'élément souple filiforme 19, matérialisé par l'une des directions de la flèche F.

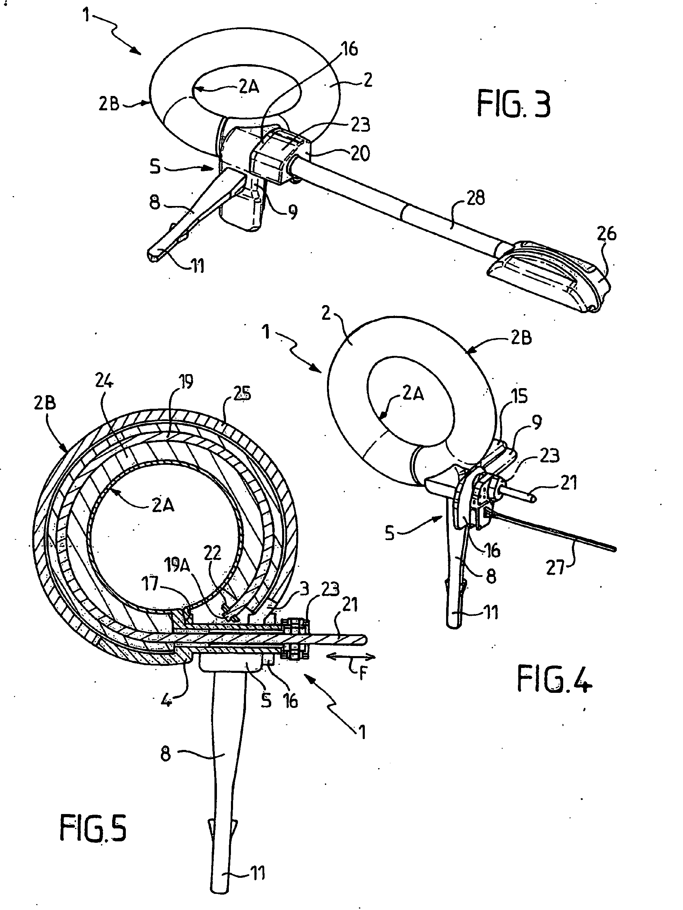

L'anneau gastrique conforme à l'invention est particulièrement conçu pour être intégré dans un système de restriction et de contrôle à distance de l'ingestion d'aliments dans l'estomac d'un patient, de telle manière que l'on puisse commander à distance, sans aucune intervention chirurgicale invasive, la variation du diamètre de l'anneau. A cette fin, l'actionneur 23 est un moteur électrique qui est avantageusement relié à un circuit de réception sous-cutané pourvu d'une antenne de réception 26 (figure 3) pour recevoir un signal radiofréquence de commande et de puissance, l'ensemble étant destiné à être implanté dans le corps du patient.

Tel qu'illustré notamment aux figures, le moteur électrique est solidarisé avec l'extrémité 4, de manière à être déporté à l'extérieur de l'anneau, le moteur électrique étant pourvu de manière classique d'un ensemble de paliers et engrenages, reliés fonctionnellement par une connexion électrique 27 au circuit de l'antenne de réception 26.

Dans cette application préférentielle, le moteur électrique est dépourvu de toute source d'alimentation interne, puisque son énergie est fournie par le circuit de réception 26, lequel convertit les ondes radio-fréquences reçues de l'unité de commande à travers l'antenne extérieure en signal de commande du moteur et en énergie pour assurer son alimentation électrique. L'antenne de réception 26 est adaptée et choisie pour recevoir à la fois un signal de commande et un signal de puissance.

Le faible besoin en énergie du moteur électrique permet d'envoyer par radiofréquence les ordres de commande et l'énergie d'actionnement du moteur évitant ainsi l'obligation d'avoir à implanter dans le corps même du patient une source additionnelle d'énergie telle qu'une pile ou une batterie.

Tel qu'illustré aux figures 3 et 4, le moteur électrique est relié à l'antenne de réception 26 par une connexion électrique 27 qui est protégée par une gaine 28 de protection assurant l'étanchéité et au bout de laquelle est montée ledit circuit de réception comportant l'antenne de réception 26. La portion libre 21 de l'élément souple filiforme 19 est également intégrée dans la gaine 28 de manière à obtenir un ensemble parfaitement protégé, étanche et susceptible d'agresser le moins possible les tissus environnants.

De manière particulièrement avantageuse, ledit circuit de l'antenne de réception 26 peut être repliable (figure 3) de manière élastique afin que le chirurgien puisse momentanément réduire les dimensions de la partie implantable du système c'est-à-dire l'anneau, la gaine 28 et le circuit de l'antenne de réception 26 pour faire passer l'ensemble monobloc dans un trocart de dimension faible, de préférence d'un diamètre inférieur par exemple à 15 mm, ceci afin de faciliter l'implantation.

Le circuit de l'antenne de réception 26 repliable sera avantageusement mais non nécessairement flexible, soit en totalité, soit au moins en partie, et formée par un circuit électrique souple, se présentant par exemple sous la forme d'un disque enrobé dans une enveloppe silicone, cette dernière servant également de protection à des composant électroniques connectés et reliés fonctionnellement à l'antenne proprement dite du circuit souple.

Selon une version particulièrement avantageuse de l'invention, le circuit de l'antenne de réception 26 se présentera sous la forme d'une pièce, par exemple en forme de disque repliable sur lui-même sensiblement selon le diamètre du disque tel que montré à la figure 3.

Grâce à cette disposition et aux propriétés d'élasticité et de souplesse des matériaux choisis, il est ainsi possible à partir de la position dépliée du circuit de l'antenne 26 de replier le circuit de l'antenne 26 le long de son diamètre pour occuper un volume restreint (figure 3), permettant son insertion dans un trocart de section circulaire.

Le système de restriction et de contrôle à distance conforme à l'invention comporte également une antenne émettrice (non représentée aux figures) disposée à l'extérieur du patient pour envoyer un signal de commande et de puissance à l'antenne de réception 26, ladite antenne émettrice étant elle-même reliée fonctionnellement à une interface de commande, tel qu'un ordinateur ou tout autre moyen équivalent à la disposition du médecin traitant.

En utilisation et une fois l'anneau gastrique conforme à l'invention implanté avec son circuit de réception muni de l'antenne 26 en position dépliée dans le corps du patient, le médecin traitant peut positionner sur la peau du patient l'antenne émettrice en position de face à face avec l'antenne de réception 26. Le médecin peut alors envoyer un signal de commande et de puissance en direction de l'antenne de réception 26, pour lui transmettre à la fois l'énergie nécessaire pour actionner l'actionneur 23 et en même temps commander son sens de déplacement.

Grâce au système de restriction et de contrôle à distance conforme à l'invention, il est ainsi possible de faire varier le diamètre de l'anneau gastrique sans avoir à pratiquer une intervention chirurgicale invasive et ce à volonté, puisque plusieurs cycles de commande peuvent être réalisés à intervalles réguliers ou non, sous la seule dépendance du médecin traitant.

Le système se révèle par ailleurs particulièrement sûr, puisque seul le médecin traitant possède le boîtier de commande comprenant l'antenne émettrice, ce qui lui permet d'exercer un contrôle total sur l'opération de réglage du diamètre. Le patient ne peut donc avoir librement accès à un moyen quelconque de réglage du diamètre de l'anneau.

L'invention concerne donc également un nouveau procédé de traitement chirurgical et thérapeutique mettant en oeuvre le système de restriction et de contrôle à distance de l'ingestion d'aliments conforme à l'invention, ainsi qu'un nouveau procédé de traitement thérapeutique mettant en oeuvre le système de fermeture de l'anneau conforme à l'invention.

On a décrit précédemment une variante préférentielle d'anneau commandable à distance, mais le système de fermeture de l'anneau conforme à l'invention peut bien sûr s'adapter à tous types d'anneaux chirurgicaux, quelles que soient leur forme, et leurs fonctionnalités (périmètre interne ou externe réglable ou non, et s'il est réglable, par quelque moyen que ce soit).

Le fonctionnement de l'anneau chirurgical conforme à l'invention est le suivant.

Après avoir introduit l'anneau 1 à l'intérieur du corps du patient à l'aide d'un trocart, le chirurgien entoure l'estomac du patient avec la bande souple 2 et rapproche la deuxième extrémité 4 de la première extrémité 3, jusqu'à ce que le premier moyen d'arrêt 16 vienne en butée contre la face arrière 7 du moyen d'encerclement 5. Le premier moyen d'arrêt 16 est maintenu naturellement plaqué contre la face arrière 7, sous l'effet de l'élasticité intrinsèque de l'anneau 1.

Le chirurgien procède ensuite à la solidarisation effective des première et deuxième extrémités 3, 4 de l'anneau, en maintenant l'élément femelle 9 et en saisissant l'extrémité libre 11 de la languette 8 formant patte de préhension, pour l'introduire à travers l'orifice 9A ménagé dans l'élément femelle 9, et exercer une traction sur la languette 8 jusqu'à ce que l'excroissance souple 8B formant came passe complètement à travers l'orifice, et vienne en butée avec sa surface d'arrêt 14 contre le bord périphérique de l'orifice 9A. Le passage de l'excroissance souple 8B est rendu possible par la souplesse de déformation de l'orifice 9A et de ladite excroissance souple 8B.

Dans le cas où une réouverture de l'anneau serait nécessaire, le chirurgien maintient l'anneau grâce à la deuxième patte de préhension 15, et exerce un effort de traction et/ou de flexion sur l'élément femelle 9, de façon à déformer l'orifice 9A et/ou l'excroissance souple 8B pour retirer la languette 8 hors de l'orifice 9A. Le chirurgien peut ainsi procéder au retrait de l'anneau 1.

## Revendications

1. - Anneau chirurgical (1) destiné à être implanté dans le corps d'un patient autour d'un organe biologique constituant une poche ou un conduit pour modifier la section de passage dudit organe lorsqu'il est enserré par l'anneau, ledit anneau (1) étant formé par une bande souple (2) comprenant une première et une deuxième extrémités (3, 4), ladite bande souple (2) étant destinée à être fermée vers ses deux extrémités (3, 4) par un système de fermeture pour former une boucle close, ladite boucle close présentant une surface interne (2A) de contact avec l'organe biologique et une surface externe opposée (2B), **caractérisé en ce que** le système de fermeture comprend un moyen d'encerclement (5) solidaire de la première extrémité (3) et agencé pour évoluer entre :
- une configuration de déverrouillage où le moyen d'encerclement (5) forme un collier ouvert libérant la deuxième extrémité (4),
- et une configuration de verrouillage où le moyen d'encerclement (5) forme un collier fermé destiné à entourer la deuxième extrémité (4) en vue de la solidariser à la première extrémité (3), ledit collier fermé présentant une face avant (6) et une face arrière (7) opposées, entre lesquelles s'étend une ouverture d'encerclement destinée à accueillir la deuxième extrémité (4).

2. - Anneau chirurgical (1) selon la revendication 1 **caractérisé en ce que** le moyen d'encerclement (5) est agencé pour passer de manière réversible entre la configuration de déverrouillage et la configuration de verrouillage.

3. - Anneau chirurgical (1) selon la revendication 1 ou 2 **caractérisé en ce que** le moyen d'encerclement (5) comprend un élément mâle (8) et un élément femelle (9), tous deux montés solidaires de la première extrémité (3) et montés sur ou relativement à cette dernière de telle façon que, lors de leur connexion mutuelle, le moyen d'encerclement (5) est verrouillé en formant le collier fermé.

4. - Anneau chirurgical (1) selon la revendication 3 **caractérisé en ce que** l'élément femelle (9) comprend un orifice (9A) traversant son épaisseur entre une première et une deuxième faces opposées (9B, 9C), tandis que l'élément mâle (8) comprend une languette destinée à être enfilée dans l'orifice (9A), ladite languette étant pourvue de moyens de blocage (8A, 8B) coopérant avec l'orifice (9A).

5. - Anneau chirurgical (1) selon la revendication 4 **caractérisé en ce que** la languette (8) présente une extrémité de liaison (10) rattachée à la surface externe de l'anneau (1) et une extrémité libre (11), l'élément femelle (9) étant également rattaché à la surface externe de l'anneau en regard de la languette (8), de façon à ce que le collier fermé s'étende vers l'extérieur de l'anneau (1).

6. - Anneau chirurgical (1) selon la revendication 4 ou 5 **caractérisé en ce que** la languette (8) comprend d'une part un premier moyen d'appui (8A) formant premier moyen de blocage et destiné à venir en appui contre le bord périphérique (13) de l'orifice (9A) sur la première face (9B) de l'élément femelle (9), et d'autre part, un deuxième moyen d'appui (8B) formant deuxième moyen de blocage et destiné à venir en appui contre le bord périphérique de l'orifice (9A) sur la deuxième face (9C) de l'élément femelle (9), ledit deuxième moyen d'appui (8B) étant conformé pour coopérer avec l'orifice (9A) à la manière d'une came avec une butée, lesdits premier et deuxième moyens d'appui (8A, 8B) étant agencés l'un relativement à l'autre pour venir, en configuration de verrouillage, enserrer entre eux l'élément femelle (9) de façon à assurer ainsi une configuration de verrouillage stable.

7. - Anneau chirurgical (1) selon la revendication 6 **caractérisé en ce que** la languette (8) présente d'une part un épaulement (8A) définissant une surface d'appui formant premier moyen d'appui, et d'autre part, une excroissance souple (8B) formant deuxième moyen d'appui, l'extrémité libre (11) de la languette (8) étant conformée pour faire office de première patte de préhension, ladite première patte de préhension permettant de faire passer la languette (8) et l'excroissance souple (8B) par l'orifice (9A) en vue de former le collier fermé.

8. - Anneau chirurgical (1) selon l'une des revendications 4 à 7 **caractérisé en ce que** la languette (8) est profilée en biseau pour faciliter son introduction et son passage dans l'orifice (9A).

9. - Anneau chirurgical (1) selon l'une des revendications 5 à 8 **caractérisé en ce que** l'anneau (1) comporte une deuxième patte de préhension (15) s'étendant à proximité de l'extrémité de liaison (10) de la languette (8), ladite deuxième patte de préhension (15) permettant de maintenir l'anneau (1) pendant l'opération de séparation des éléments mâle (8) et femelle (9), réalisée en vue d'ouvrir l'anneau (1).

10. -Anneau chirurgical (1) selon l'une des revendications 5 à 9 **caractérisé en ce que** l'élément femelle (9) comprend une troisième patte de préhension (9) permettant la séparation des éléments mâle et femelle, en vue d'ouvrir l'anneau (1).

11. -Anneau chirurgical (1) selon l'une des revendications précédentes **caractérisé en ce que** la deuxième extrémité (4) de l'anneau (1) est pourvue d'un premier moyen d'arrêt (16) destiné à venir en butée contre la face arrière (7) du collier fermé entourant la deuxième extrémité (4) de l'anneau en configuration de verrouillage, de façon à empêcher le déplacement de la deuxième extrémité (4) dans le sens de l'ouverture de l'anneau (1).

12. -Anneau chirurgical (1) selon l'une des revendications précédentes **caractérisé en ce que** la deuxième extrémité (4) de l'anneau (1) est pourvue d'un deuxième moyen d'arrêt (17) destiné à venir en butée contre la face avant (6) du collier fermé entourant la deuxième extrémité (4) de l'anneau (1) en configuration de verrouillage, de façon à empêcher le déplacement de la deuxième extrémité (4) dans le sens de la fermeture de l'anneau (1).

13. -Anneau chirurgical (1) selon les revendications 10 et 11 **caractérisé en ce que** lesdits premier et deuxième moyen d'arrêt (16, 17) sont agencés l'un relativement à l'autre pour enserrer entre eux le collier fermé (5) en configuration de verrouillage, de façon à sensiblement empêcher tout déplacement de la deuxième extrémité (4) relativement à la première extrémité (3).

14. -Anneau chirurgical (1) selon l'une des revendications précédentes **caractérisé en ce que** la bande souple (2) présente une portion de section réduite (18) au niveau de la deuxième extrémité (4) de l'anneau (1), ladite portion (18) étant destinée à venir se loger latéralement dans une échancrure (19B) de forme sensiblement complémentaire ménagée au niveau de la première extrémité (3), ladite échancrure (19) formant une partie du collier fermé en configuration de verrouillage, de façon à assurer une continuité de la surface interne (2A) de l'anneau (1).

15. -Anneau chirurgical (1) selon l'une des revendications 11 à 13 et selon la revendication 14 **caractérisé en ce que** la bande souple (2) présente un épaulement (17) au niveau de la transition à la portion de section réduite (18), ledit épaulement (17) faisant office de deuxième moyen d'arrêt.

16. -Anneau (1) selon l'une des revendications précédentes **caractérisé en ce que** la bande souple (2) et le système de fermeture (5) forment une pièce monobloc réalisée à partir du même matériau.

17. -Anneau (1) selon l'une des revendications précédentes **caractérisé en ce qu**'il comprend un système (19, 23, 26, 27, 28) pour commander de manière réversible la variation de son périmètre interne, ledit système (19, 23, 26, 27, 28) comportant un élément souple filiforme (19) inséré longitudinalement et à coulissement dans le matériau (24) formant le corps de l'anneau (1) sensiblement entre les première et deuxième extrémités (3, 4), pour définir une portion fixe (19A) solidarisée avec la première extrémité (3) et une portion libre (21) associée fonctionnellement à un actionneur (23) monté sur l'anneau (1), de telle manière que l'actionneur (23) puisse assurer la translation réversible de l'élément souple filiforme (19) pour obtenir une variation associée du diamètre de l'anneau (1).

18. -Anneau (1) selon la revendication 17 lorsqu'elle dépend de la revendication 11 **caractérisé en ce que** l'actionneur (23) est agencé sur l'anneau (1) pour constituer le premier moyen d'arrêt (16) ou être associé à celui-ci.

19. -Anneau (1) selon l'une des revendications précédentes **caractérisé en ce qu'**il est formé par un anneau gastrique destiné à être implanté autour de l'estomac ou de l'oesophage.

20. -Anneau (1) selon l'une des revendications 1 à 18 **caractérisé en ce qu**'il est formé par un anneau destiné à être implanté autour de la vessie ou des voies urinaires ou autour des voies gastro-intestinales ou autour de vaisseaux sanguins.

## Claims

1. Surgical ring (1) for being implanted into the body of a patient around a biological organ constituting a pocket or a conduit for modifying the passing section of the organ when it is constricted by the ring, said ring (1) being formed by a flexible belt (2) comprising first and second extremities (3, 4), said flexible belt (2) being adapted to be closed at its two extremities (3, 4) by a closure system for forming a closed loop, the closed loop having an internal contact surface (2A) with the biological organ and an opposed external surface (2B), **characterized in that** the closure system comprises an encircling means (5) which is integral with the first extremity (3) and movable between:
- an opened configuration wherein the encircling means (5) forms an open collar releasing the second extremity (4),
- and a locked configuration wherein the encircling means (5) forms a closed collar for encircling the second extremity (4) in order to connect it to the first extremity (3), said closed collar having a front face (6) and an opposed back face (7) with an encircling opening for receiving the second extremity (4) extending between the front and back face.

2. Surgical ring (1) according to claim 1, **characterized in that** the encircling means (5) is arranged to reversibly switch between the opened and the locked configuration.

3. Surgical ring (1) according to claim 1 or 2, **characterized in that** the encircling means (5) comprises a male element (8) and a female element (9), both mounted integrally to the first extremity (3) and mounted on or relatively to this extremity so that, at their mutual connection, the encircling means (5) is locked by forming the closed collar.

4. Surgical ring (1) according to claim 3, **characterized in that** the female element (9) comprises a through hole (9A) between first and second opposed faces (9B, 9C), while the male element (8) comprises a strap for being fed into the through hole (9A), said strap having blocking means (8A, 8B) cooperating with the through hole (9A).

5. Surgical ring (1) according to claim 4, **characterized in that** the strap (8) has a linking extremity (10) attached to the external surface of the ring (1) and a free extremity (11), wherein the female element (9) is also attached to the external surface of the ring vis-a-vis the strap (8), so that the closed collar extends towards the exterior of the ring (1).

6. Surgical ring (1) according to claim 4 or 5, **characterized in that** the strap (8) comprises, on the one hand, a first support means (8A) forming the first blocking means and designated for abutting the peripheral side (13) of the through hole (9A) on the first face (9B) of the female element (9), and, on the other hand, a second support means (8B) forming the second blocking means and designated for abutting the peripheral side of the through hole (9A) on the second face (9C) of the female element (9), said second support means (8B) being adapted to cooperate with the through hole (9A) like a cam with an abutment, said first and second support means (8A, 8B) being arranged, in a locked configuration, to receive the female element (9) between them in order to assure a stable locked configuration.

7. Surgical ring (1) according to claim 6, **characterized in that** the strap (8) comprises, on the one hand, a ledge (8A) defining a support surface forming first support means, and, on the other hand, a flexible excrescence (8B) forming second support means, the free extremity (11) of the strap (8) being adapted to act as the first gripper, said first gripper allowing to pass the strap (8) and the flexible excrescence (8B) through the through hole (9A) in order to form a closed collar.

8. Surgical ring (1) according to any one of claims 4 to 7, **characterized in that** the strap (8) is bevelled in order to facilitate its introduction and its passage in the through hole (9A).

9. Surgical ring (1) according to any one of claims 5 to 8, **characterized in that** the ring (1) comprises a second gripper (15) extending near the linking extremity (10) of the strap (8), wherein said second gripper (15) allows to maintain the ring (1) during the separation operation of the male (8) and female (9) elements carried out in order to open the ring (1).

10. Surgical ring (1) according to any one of claims 5 to 9, **characterized in that** the female element (9) comprises a third gripper (9) allowing the separation of the male and female elements, in order to open the ring (1).

11. Surgical ring (1) according to any one of the preceding claims, **characterized in that** the second extremity (4) of the ring (1) is equipped with a first stop means (16) for abutting the back face (7) of the closed collar receiving the second extremity (4) of the ring in the locked configuration, so as to avoid the displacement of the second extremity (4) in the opening direction of the ring (1).

12. Surgical ring (1) according to any one of the preceding claims, **characterized in that** the second extremity (4) of the ring (1) is equipped with a second stop means (17) for abutting the front face (6) of the closed collar receiving the second extremity (4) of the ring (1) in the locked configuration, so as to avoid the displacement of the second extremity (4) in the closing direction of the ring (1).

13. Surgical ring (1) according to claims 10 and 11, **characterized in that** said first and second stop means (16, 17) are assembled relative to each other in order to receive between them the closed collar (5) in locked configuration, so as to substantially avoid any displacement of the second extremity (4) vis-a-vis the first extremity (3).

14. Surgical ring (1) according to any preceding claim, **characterized in that** the flexible belt (2) has a reduced cross-section portion (18) near the second extremity (4) of the ring (1), said portion (18) being designated to fit laterally in a notch (19B) of substantially complementary shape provided on the area of the first extremity (3), said notch (19) forming a part of the closed collar in the locked configuration, so as to assure a continuity of the internal surface (2A) of the ring (1).

15. Surgical ring (1) according to any one of claims 11 to 13 and according to claim 14, **characterized in that** the flexible belt (2) has a ledge (17) in the area of the transition to the reduced cross-section portion (18), said ledge (17) serving as second stop means.

16. Ring (1) according to any one of the preceding claims, **characterized in that** the flexible belt (2) and the closure system (5) form a monobloc piece made from the same material.

17. Ring (1) according to any one of the preceding claims, **characterized in that** it comprises a system (19, 23, 26, 27, 28) for reversibly controlling the variation of its internal perimeter, said system (19, 23, 26, 27, 28) comprising a flexible filament-shaped element (19) which is longitudinally inserted and sliding in the material (24) forming the body of the ring (1) substantially between the first and second extremities (3, 4) in order to define a fixed portion (19A) connected to the first extremity (3) and a free portion (21) functionally associated to an operating gear (23) installed on the ring (1), so that the operating gear (23) allows the reversible displacement of the flexible filament-shaped element (19) in order to obtain an associated variation of the diameter of the ring (1).

18. Ring (1) according to claim 17 as far as it depends on claim 11, **characterized in that** the operating gear (23) is assembled to the ring (1) for constituting the first stop means (16) or being associated to the same.

19. Ring (1) according to any one of the preceding claims, **characterized in that** it is formed by a gastric ring for being implanted around the stomach or the esophagus.

20. Ring (1) according to any one of claims 1 to 18, **characterized in that** it is formed by a ring for being implanted around the bladder or the urinary passages or around gastrointestinal passages or around blood vessels.

## Patentansprüche

1. Chirurgischer Ring (1), der in den Körper eines Patienten um ein eine Tasche oder einen Kanal bildendes biologisches Organ herum zu implantieren ist, um den Durchlaßquerschnitt des Organs zu modifizieren, wenn es von dem Ring umschlossen ist, wobei der Ring (1) von einem biegsamen Band (2) gebildet wird, das ein erstes und ein zweites Ende (3,4) aufweist, wobei das biegsame Band (2) mittels eines Schließsystems zu seinen zwei Enden (3,4) hin zu schließen ist, um eine geschlossene Schlaufe zu bilden, wobei die geschlossene Schlaufe eine innere Oberfläche (2A) des Kontakts mit dem biologischen Organ und eine gegenüberliegende äußere Oberfläche (2B) aufweist, **dadurch gekennzeichnet, daß** das Schließsystem eine Umklammerungseinrichtung (5) aufweist, die mit dem ersten Ende (3) integral ist und sich bewegen läßt zwischen:
- einer Entriegelungskonfiguration, in welcher die Umklammerungseinrichtuhg (5) eine das zweite Ende (4) freigebende offene Schelle bildet, und
- einer Verriegelungskonfiguration, in welcher die Umklammerungseinrichtung (5) eine geschlossene Schelle bildet, die das zweite Ende (4) umgibt, um es mit dem ersten Ende (3) zu verbinden, wobei die geschlossene Schelle eine vordere Fläche (6) und eine hintere Fläche (7) aufweist, die einander gegenüberliegen und zwischen denen sich eine Umklammerungsöffnung erstreckt, um das zweite Ende (4) aufzunehmen.

2. Chirurgischer Ring (1) nach Anspruch 1, **dadurch gekennzeichnet, daß** sich die Umklammerungseinrichtung (5) auf reversible Weise zwischen der Entriegelungskonfiguration und der Verriegelungskonfiguration ändern läßt.

3. Chirurgischer Ring (1) nach Anspruch 1 oder 2, **dadurch gekennzeichnet, daß** die Umklammerungseinrichtung (5) ein männliches Element (8) und ein weibliches Element (9) aufweist, die alle beide mit dem ersten Ende (3) integral sind und auf eine Weise an dem ersten Ende öder bezüglich des ersten Endes angebracht sind, daß, wenn sie miteinander verbunden sind, die Umklammerungseinrichtung (5) verriegelt ist und eine geschlossene Schelle bildet.

4. Chirurgischer Ring (1) nach Anspruch 3, **dadurch gekennzeichnet, daß** das weibliche Element. (9) eine Öffnung (9A) aufweist, die durch seine Dicke zwischen einander gegenüberliegenden ersten und zweiten Flächen (9B,9C) hindurchgeht, während das männliche Element (8) eine Zunge aufweist, die in die Öffnung (9A) einzufädeln ist, wobei die Zunge mit Sperreinrichtungen (8A, 8B) versehen ist, die mit der Öffnung (9A) zusammenwirken.

5. Chirurgischer Ring (1) nach Anspruch 4, **dadurch gekennzeichnet, daß** die Zunge (8) ein an der äußeren Oberfläche des Rings (1) angebrachtes Verbindungsende (10) und ein freies Ende (11) aufweist, wobei das weibliche Element (9) ebenfalls an der äußeren Oberfläche des Rings gegenüber der Zunge (8) angebracht ist, derart, daß sich die geschlossene Schelle von dem Ring (1) aus auswärts erstreckt.

6. Chirurgischer Ring (1) nach Anspruch 4 oder 5, **dadurch gekennzeichnet, daß** die Zunge (8) einerseits eine erste Abstützungseinrichtung (8A) aufweist, die eine erste Sperreinrichtung bildet und an dem peripheren Rand (13) der Öffnung (9A) an der ersten Fläche (9B) des weiblichen Elements (9) zur Abstützung kommt, und andererseits eine zweite Abstützungseinrichtung (8B) aufweist, die eine zweite Sperreinrichtung bildet und an dem peripheren Rand der Öffnung (9A) an der zweiten Fläche (9C) des weiblichen Elements (9) zur Abstützung kommt, wobei die zweite Abstützungseinrichtung (8B) derart gestaltet ist, daß sie mit der Öffnung (9A) wie ein Nocken mit einem Anschlag zusammenwirken kann, wobei die erste und zweite Abstützungseinrichtung (8A, 8B) so zueinander angeordnet sind, um in der Verriegelungskonfiguration das weibliche Element (9) zwischen sich einzuschließen, um auf diese Weise eine stabile Verriegelungskonfiguration sicherzustellen.

7. Chirurgischer Ring (1) nach Anspruch 6, **dadurch gekennzeichnet, daß** die Zunge (8) einerseits eine Schulter (8A) aufweist, die eine Abstützungsfläche definiert, die eine erste Abstützungseinrichtung bildet, sowie andererseits einen biegsamen Vorsprung (8B) aufweist, der die zweite Abstützungseinrlchtung bildet, wobei das freie Ende (11) der Zunge (8) gestaltet ist, um als.erste Greifklaue zu dienen, wobei die erste Greifklaue erlaubt, die Zunge (8) und den biegsamen Vorsprung (8B) durch die Öffnung (9A) hindurch zu führen, um die geschlossene Schelle zu bilden.

8. Chirurgischer Ring (1) nach einem der Ansprüche 4 bis 7, **dadurch gekennzeichnet, daß** die Zunge (8) ein abgeschrägtes Profil hat, um ihre Einführung in und ihren Durchgang durch die Öffnung (9A) zu erleichtern.

9. Chirurgischer Ring (1) nach einem der Ansprüche 5 bis 8, **dadurch gekennzeichnet, daß** der Ring (1) eine zweite Greifklaue (15) aufweist, die sich in der Nähe des Verbindungsendes (10) der Zunge (8) erstreckt, wobei die zweite Greifklaue (15) erlaubt, den.Ring (1) während der Trennung des männlichen (8) und des weiblichen Elements (9) zu halten, die durchgeführt wird, um den Ring (1) zu öffnen.

10. Chirurgischer Ring (1) nach einem der Ansprüche 5 bis 9, **dadurch gekennzeichnet, daß** das weibliche Element (9) eine dritte Greifklaue (9) aufweist, die zum Öffnen des Rings das Trennen des männlichen und weiblichen Elements erlaubt.

11. Chirurgischer Ring (1) nach einem der vorstehenden Ansprüche, **dadurch gekennzeichnet, daß** das zweite Ende (4) des Rings (1) mit einer Anschlageinrichtung (16) versehen ist, die in der Verriegelungskonfiguration an der hinteren Fläche (7) der das zweite Ende (4) des Rings umgebenden geschlossenen Schelle zum Anschlag kommt, um zu verhindern, daß sich das zweite Ende (4) im Sinne eines Öffnens des Rings (1) verschiebt.

12. Chirurgischer Ring (1) nach einem der vorstehenden Ansprüche, **dadurch gekennzeichnet, daß** das zweite Ende (4) des Rings (1) mit einer zweiten Anschlageinrichtung (17) versehen ist, die in der Verriegelungskonfiguration an der vorderen Fläche (6) der das zweite Ende (4) des Rings (1) umgebenden geschlossenen Schelle zum Anschlag kommt, um zu verhindern, daß sich das zweite Ende (4) im Sinne eines Schließens des Rings (1) verschiebt.

13. Chirurgischer Ring (1) nach Anspruch 10 und 11, **dadurch gekennzeichnet, daß** die erste und die zweite Anschlageinrichtung (16, 17) so zueinander angeordnet sind, um in der Verriegelungskonfiguration die geschlossene Schelle (5) zwischen sich einzuschließen, um jegliche Verschiebung des zweiten Endes (4) relativ zu dem ersten Ende (3) wesentlich zu verhindern.

14. Chirurgischer Ring (1) nach einem der vorstehenden Ansprüche, **dadurch gekennzeichnet, daß** das biegsame Band (2) im Bereich des zweiten Endes (4) des Rings (1) einen im Querschnitt reduzierten Abschnitt (18) aufweist, wobei der Abschnitt (18) seitlich in einer an dem ersten Ende (3) ausgesparten, im wesentlichen komplementär gestalteten Aussparung (19B) aufzunehmen ist, wobei in der Verriegelungskonfiguration die Aussparung (19) einen Teil der geschlossenen Schelle bildet, um eine Kontinuität der inneren Oberfläche (2A) des Rings (1) sicherzustellen.

15. Chirurgischer Ring (1) nach einem der Ansprüche 11 bis 13 und nach Anspruch 14, **dadurch gekennzeichnet, daß** das biegsame Band (2) am Ort des Übergangs zu dem im Querschnitt reduzierten Abschnitt (18) eine Schulter (17) aufweist, die als zweite Anschlageinrichtung dient.

16. Ring (1) nach einem der vorstehenden Ansprüche, **dadurch gekennzeichnet, daß** das biegsame Band (2) und das Schließsystem (5) einen einstückigen Block bilden, der ausgehend von dem gleichen Material hergestellt ist.

17. Ring (1) nach einem der vorstehenden Ansprüche, **dadurch gekennzeichnet, daß** er ein System (19, 23, 26, 27, 28) zur Steuerung der reversiblen Änderung seines Innendurchmessers aufweist, wobei das System (19, 23, 26, 27, 28) ein biegsames fadenförmiges Element (19) aufweist, das längs und gleitbar in dem den Körper des Rings (1) bildenden Material (24) im wesentlichen zwischen dem ersten und dem zweiten Ende (3,4) eingesetzt ist, um einen mit dem ersten Ende (3) verbundenen fixierten Abschnitt (19A) und einen freien Abschnitt (21) zu definieren, der funktionell mit einem an dem Ring (1) angebrachten Betätigungsorgan (23) verbunden ist, so daß das Betätigungsorgan (23) die reversible Translation des fadenförmigen, biegsamen Elements (19) sicherstellen kann, um eine entsprechende Änderung des Durchmessers des Rings (1) zu erzielen.

18. Ring (1) nach.Anspruch 17, soweit abhängig von Anspruch 11, **dadurch gekennzeichnet, daß** das Betätigungsorgan (23) an dem Ring (1) angeordnet ist, um die erste Anschlageinrichtung (16) zu bilden oder ein Teil derer zu sein.

19. Ring (1) nach einem der vorstehenden Ansprüche, **dadurch gekennzeichnet, daß** er von einem Magenring gebildet wird, der konzipiert ist, um den Magen oder die Speiseröhre herum implantiert zu werden.

20. Ring (1) nach einem der Ansprüche 1 bis 18, **dadurch gekennzeichnet, daß** er von einem Ring gebildet wird, der konzipiert ist, um die Harnblase oder die Harnwege herum oder um Magen-Darm-Wege herum oder um Blutgefäße herum implantiert zu werden.
